# EUROPÄISCHE PATENTSCHRIFT

(11) **EP 1 776 968 B1**
(45) Veröffentlichungstag und Bekanntmachung des Hinweises auf die Patenterteilung: **04.09.2013**
(21) Anmeldenummer: 06021169.5
(22) Anmeldetag: 10.10.2006
(51) Int. Cl.: A61L 27/26, A61L 27/54, H04R 25/00, G03F 7/027

(54) **Niedrigviskose, strahlungshärtbare Formulierung zur Herstellung von Ohrpassstücken mit antimikrobiellen Eigenschaften**
Low-viscosity radiation-curable formulation for the preparation of ear pieces with antimicrobial properties
Formulation durcissable par irradiation et à basse viscosité pour la préparation d'embouts auriculaires avec des propriétés antimicrobiennes

(30) Priorität: 18.10.2005 DE 102005050186
(43) Veröffentlichungstag der Anmeldung: 25.04.2007
(73) Patentinhaber: Dreve Otoplastik GmbH, 59423 Unna (DE)
(72) Erfinder: Klare, Martin, Dr., 44227 Dortmund (DE); Altmann, Reiner, Dr., 44575 Castrop-Rauxel (DE); Kutschinski, Michael, 44579 Castrop-Rauxel (DE); Veit, Thomas, Dr., 48151 Münster (DE)
(74) Vertreter: Köchling, Conrad-Joachim

(56) Entgegenhaltungen:
- WO-A1-2005/001570
- WO-A2-03/072623

## Beschreibung

Die vorliegende Erfindung betrifft niedrigviskose, strahlungshärtbare Formulierungen zur Herstellung von medizintechnischen Produkten mit antimikrobiellen Eigenschaften, insbesondere zur Herstellung von Ohrpassstücken, die mittels des PNP-Verfahrens (U. Voogdt, "Otoplastik" 2 Aufl., Bd.2, Median-Verlag von Killisch-Horn GmbH, S.22 ff (1998)) oder eines regenerativen Verfahrens wie zum Beispiel der Stereolithographie hergestellt werden, auf Basis mindestens zweier Verbindungen, die radikalisch polymerisierbare (Meth)acrylatfunktionen aufweisen, und mindestens eines für die Polymerisation der geeigneten Verbindungen geeigneten Photoinitiators, und mindestens eines für die Stabilisisierung der Formulierungen bestimmten Inhibitors, und mindestens einer oder einer Kombination von antimikrobiell bzw. bakteriostatisch wirkenden Komponenten, die charakterisiert dadurch sind, dass es sich dabei entweder um einen bioaktiven Glasfüller, um silberionenfreisetzende, nanoskalige Silberpulver oder eine polymerisierbare Verbindung mit antimikrobiellen Eigenschaften handelt.

Beim so bezeichneten PNP-Verfahren (Positiv-Negativ-Positiv) nimmt der Hörgeräteakustiker in einem ersten Schritt einen Ohrabdruck (Positiv) zur Herstellung einer Otoplastik (für hinter dem Ohr getragene Geräte) oder einer Schale (für im Ohr getragene Geräte). In einem zweiten Schritt wird mittels der Abformung eine Negativform (N) angefertigt, in die nachfolgend die strahlungshärtbare, niedrigviskose Formulierung gegossen und daraufhin belichtet wird. Das so gefertigte Ohrpassstück (Positiv) muss dem Gehörgang optimal angepasst sein. Andernfalls würden ungenaue Passstücke Beschwerden (zum Beispiel Druckstellen) verursachen und die Funktion von Hörgeräten (z. B. Rückkopplungen) beeinträchtigen. Demzufolge ist es wichtig, dass die Formulierung möglichst niedrigviskos dass heißt, "gut fließend" ist, so dass auch Unterschnitte und feinste Oberflächentexturen vom Material ausgefüllt und so abgebildet werden können.

Es ist aus US. Pat. 4,575,330 bekannt, dass niedrigviskose, strahlungshärtbare Harze beziehungsweise Harzgemische für die Herstellung von dreidimensionalen Objekten mittels Stereolithographie eingesetzt werden können. Ferner ist aus US. Pat. 5,487,012 und WO 01/87001 bekannt, dass die Stereolithographie vorteilhaft zur Herstellung von Ohrstücken eingesetzt werden kann. Beim stereolithographischen Verfahren werden dreidimensionale Objekte aus einer niedrigviskosen, strahlungshärtbaren Formulierung in der Weise aufgebaut, dass jeweils eine dünne Schicht (ca. 0,0025-0,1 mm) der Formulierung mittels aktinischer Strahlung in definierter Weise so vorhärtet, dass die erzeugte Schicht die gewünschte Querschnittsform des Objektes an dieser Stelle vorweist. Zeitgleich wird die erzeugte Schicht an die im Schritt zuvor gehärtete Schicht polymerisiert. Der Aufbau des Gesamtobjektes lässt sich so mit Hilfe eines computergesteuerten Lasersystems wie zum Beispiel, eines Nd:YVO₄ Festkörperlasers (Viper si² SLA System, Fa. 3D Systems, USA) bewerkstelligen. Der generierte Formkörper wird gegebenenfalls, zum Beispiel durch Strahlung, nachgehärtet.

An die im stereolithographischen Prozess einsetzbaren Harzformulierungen werden besondere Anforderungen gestellt. Dabei sind insbesondere die Strahlungsempfindlichkeit und die Viskosität der Harzformulierungen, sowie die Festigkeit der mittels Laserhärtung vorgehärteten Formkörper zu nennen. Dieser nicht völlig gehärtete Formkörper wird in der Technik der Stereolithographie als Grünling bezeichnet, und die Festigkeit dieses Grünlings, charakterisiert durch den E-Modul und die Biegefestigkeit, bezeichnet man als Grünfestigkeit. Die Grünfestigkeit stellt für die Praxis der Stereolithographie einen wichtigen Parameter dar, da Formkörper mit geringer Grünfestigkeit sich während des Stereolithographieprozesses unter ihrem eigenen Gewicht deformieren oder während der Nachhärtung, beispielsweise mit einer Xenonbogen- oder Halogenlampe, absacken oder sich durchbiegen können. Es ist daher verständlich, dass unter Berücksichtigung der oben genanten Anforderungen kompliziert abgestimmte und formulierte Zusammensetzungen eingesetzt werden.

Beispielsweise wird von H. Kodama in Rev. Sci. Instrum. 52 (11), 1170-1173 (1981) eine niedrigviskose, strahlungshärtbare Harzformulierung offenbart, die aus einem ungesättigten Polyester, einem Acrylsäureester, Styrol und einem Polymerisationsinitiator besteht. Im Hinblick auf den Einsatz in der Stereolithographietechnik weist diese Harzformulierung jedoch eine geringe Grünfestigkeit und eine ungenügende Photoempfindlichkeit auf.
Eine ebenfalls unter produktionstechnischen Gesichtspunkten ungenügende Photoempfindlichkeit weisen die in US. Pat. 4,100,141 offenbarten Harzformulierungen auf. Dabei bedeutet eine geringe Photoempfindlichkeit, dass lange Zeiten für die Herstellung der Formkörper benötigt werden. Dementsprechend muss die Photoempfindlichkeit der Stereolithographieharzformulierungen so eingestellt sein, dass sich aus dem Verhältnis von erzielter Eindringtiefe des Laserstrahles in die niedrigviskose, strahlungshärtbare Harzformulierung und der aufgewandten Strahlungsenergie ergibt, dass mit geringer Strahlungsenergie eine möglichst große Härtungstiefe bei gleichzeitig hohem Polymerisationsgrad, guter Grünfestigkeit und ausreichender Stabilität der Harzformulierung gegen Autopolymerisation realisiert werden. Flüssige, strahlungshärtbare Harzformulierungen die den o. g. Anforderungen teilweise genügen sind beispielsweise in US. Pat. 5,476,748 oder in WO 99/50711 beschrieben. Diese als "Hybrid-System" bezeichneten Formulierungen enthalten allerdings eine Kombination aus radikalisch und kationisch polymerisierbaren Komponenten. Sie bestehen erstens aus einer flüssigen, di- bzw. polyfunktionellen Epoxyverbindung oder aus einer Mischung, bestehend aus difunktionellen oder höherfunktionellen Epoxyverbindungen; zweitens aus einem kationischem Photoinitiator beziehungsweise aus einer Mischung von kationischen Photoinitiatoren; drittens aus einem Photoinitiator beziehungsweise aus einer Mischung von Photoinitiatoren für die freie radikalische Polymerisation und mindestens aus einem niedrigviskosen Poly(meth)acrylat mit einer (Meth)acrylatfunktionalität von n>2, mindestens einem Diacrylat und einer Polyolkomponente aus der Gruppe hydroxylterminierter Polyether, Polyester und Polyurethane. Dem Fachmann ist bekannt, dass solche Formulierungen unter toxikologischen Aspekten kritisch zu bewerten sind und demzufolge nicht oder nur eingeschränkt zur Herstellung von Medizinprodukten eingesetzt werden können. Beispielsweise ist bekannt, dass kationische Photoinitiatoren zu Hautreizungen und allergischen Reaktionen führen können. Ähnliches gilt für die in solchen Formulierungen eingesetzten Verbindungen mit Epoxidfunktionen. Es ist dem Fachmann ferner bekannt, dass viele Acrylatverbindungen - insbesondere kurzkettige- ebenfalls ein erhöhtes Allergiepotential besitzen und somit Harzzusammensetzungen, wie sie z.B. in EP 0425441,
EP 0914242 und EP 0579503 beschrieben sind, unter Biokompatibilitätsaspekten nicht zur Herstellung von z. b. Ohrstücken verwendet werden können. Für den Einsatz in der Medizintechnik haben sich monomere oder oligomere Dimethacrylate auf Basis von Bisphenol A oder Bisphenol F und Urethanmethacrylate mit einer Funktionalität von n ≥ 2 bewährt. Im Vergleich zur Gruppe der Acrylatverbindungen weist für die Technik der Stereolithographie die Gruppe der Methacrylatverbindungen jedoch eine geringere Reaktivität auf. Daraus ergeben sich die schon o. g. Nachteile hinsichtlich Eindringtiefe des Laserstrahles und Grünfestigkeit der vorgehärteten Objekte. Darüber hinaus sind aufgrund der verminderten Reaktivität dieser Verbindungsklasse höhere Konzentrationen an einem oder mehreren Photoinitiatoren für die freie radikalische Polymerisation einzusetzen. Daraus resultiert eine verringerte Stabilität gegen Autopolymerisation der Harzformulierung. Dem Fachmann ist weiterhin bekannt, dass in der Technik der Stereolithographie bei der Herstellung einer großen Anzahl kleinteiliger Objekte von geringer Masse eine erhöhte mechanische und thermische Belastung der Stereolithographieharzformulierung vorliegt, die zu einer Autopolymerisation des Stereolithographieharzes bzw. zu sich verändernden Eigenschaften der Harzzusammensetzung und der daraus generierten Formkörper führen kann. Dies ist zum einen darauf zurückzuführen, dass bei geringem Harzverbrauch die auf einer Plattform fixierten, vorgehärteten Formkörper relativ häufig aus dem Bauraum der Stereolithographieanlage entfernt werden müssen. Daraus resultieren Temperaturschwankungen des Stereolithographieharzes im Bauraum. Zum anderen ergibt sich bei der Produktion von Ohrstücken ein relativ großes Oberflächen- zu Volumenverhältnis der generierten Formkörper. Es ist dem Fachmann bekannt, dass bei der freien radikalischen Polymerisation infolge von Sauerstoffzutritt eine Inhibierungsschicht an der Oberfläche der Formkörper zurückbleibt. Das nur unvollständig an- bzw. auspolymerisierte Harz kann sich so während des Bauprozesses von der Oberfläche des Probenkörpers in das Stereolithographieharz lösen.
Ein weiterer wichtiger Punkt für solche Formulierung ist die Flexibilität der erhaltenen Formkörper. Dem Fachmann ist bekannt, dass die kommerziell erhältlichen Formulierungen, die für die o. g. Anwendungen ausreichende Biokompatibilität aufweisen, spröde sind. Die durchschnittlichen Dehnungswerte für ausgehärtete Objekte liegen zwischen 4-8% (DIN EN ISO 178). Bei erhöhter mechanischer Belastung kann es so zu einem Bruch der generierten Ohrpassstücke kommen, der zu scharfkantigen Bruchteilen führen kann. Dies ist im Hinblick auf eine erhöhte Verletzungsgefahr unerwünscht. Zur Lösung dieser Problematik sind verschiedene Strategien, wie der Einsatz monofunktioneller Verdünnermonomere (JP 97-431498) oder der Einsatz von monomeren beziehungsweise oligomeren, niedrigviskosen Urethanacrylaten (DE 4,138,309) verfolgt worden. Durch Einsatz der genannten Komponenten können Werkstoffe mit erhöhter Flexibilität realisiert werden. Allerdings ergeben sich durch die o. g. Formulierungskomponenten Nachteile wie ein erhöhter Schrumpf, eine erhöhte Wasseraufnahme und durch niedrigviskose Urethanacrylate ein erhöhtes allergiesierendes Potential. Darüber hinaus ist der Einsatz von Polyetherpolyolen (WO 97/38354) zur Erniedrigung der Vernetzungdichte des 3-dimensionalen Polymernetzwerkes beschrieben, um die Sprödigkeit von Formkörpern zu erniedrigen. Zu den Nachteilen dieser Methode zählen aber ein Verlust an Festigkeit und eine wesentliche Verringerung der Wasser- und Feuchtigkeitsstabilität der generierten Formkörper.
Aus diesen Punkten ist ersichtlich, dass bei der Herstellung von Ohrstücken mittels des PNP-Verfahrens und der stereolithographischen Technik die Minimierung der Viskosität der strahlungshärtbaren Harzzusammensetzung unter Beibehaltung von Biokompatibilität und akzeptabler chemisch-physikalischer, sowie mechanischer Eigenschaften der generierten Formkörper, ein bedeutender Parameter ist. Unter produktionstechnischen Gesichtspunkten mittels Stereolithographie ist es weiterhin wünschenswert, dass sowohl die Eindringtiefe des Laserstrahles und die kritische Energie der Stereolithographieharzformulierung der Anwendung entsprechend eingestellt werden kann. Die so gefertigten Ohrpassstücke werden heutzutage sowohl aus ästhetischen als auch aus Gründen des Tragekomforts und der Reinigung in vielen Fällen beschichtet. Es kommen dabei im Wesentlichen Beschichtungen auf Methylmethacrylatbasis, Vergoldungen oder mittels Sol-Gel-Technik hergestellte Systeme, wie sie in DE 10219679 beschrieben sind, zum Einsatz. Letztere werden z. B. von der Fa. Audio Service kommerziell vertrieben. Die generierten Ohrpassstücke stehen in unmittelbaren Kontakt mit der Haut des Ohres, wobei das feuchte und warme "Klima" im Gehörganges nahezu ideale Voraussetzungen für das Wachstum von Bakterien und Pilzen bietet. Kommt es nun zu Druckstellen durch das Ohrpassstück oder zu Reibungen zwischen diesem und der Haut infolge von zum Beispiel Kaubewegungen, so können Entzündungen die Folge sein. Es ist deshalb wünschenswert, die Beschichtungen antibakteriell auszurüsten. Eine kommerzielle Lösung, die auf der bakteriziden und fungiziden Wirkung von Silber basiert, wird von der Fa. Audioservice unter dem Namen ComforMed angeboten. Der Beschichtungsvorgang ist jedoch mit hohem manuellen, apparativen und somit auch Kostenaufwand verbunden. Darüber hinaus wird durch das Aufbringen einer Beschichtung, die Dicke der Lackschichten liegen bei ca. 20 bis 60 µm, die Passform des generierten Ohrpassstückes nachhaltig verändert. Dementsprechend muss die Schichtdicke der Beschichtung schon bei der Herstellung des Ohrpassstückes durch ein virtuelles Offset mit berücksichtigt werden. Zur Vermeidung dieser aufwändigen Produktionsschritte ist es daher wünschenswert, ein Material zur Herstellung von Ohrpassstücken bereitzustellen, welches einerseits antimikrobiell ausgestattet ist und dementsprechend keiner Beschichtung bedarf, und andererseits beim PNP-Verfahren und insbesondere bei den o. g. generativen Herstellverfahren zu möglichst homogenen und glatten Oberflächen führt.

Aus der WO03/072623 A ist eine härtbare Zusammensetzung und ein Verfahren zur Herstellung einer weichen, aber stabilen Hörhilfe bekannt. Die dortige Zusammensetzung beinhaltet ein Urethan-Acrylatoligmer, einen reaktiven Weichmacher, also ein monofunktionelles (Meth)arcylat, einen Photoinitiator und Farbstoff.

Die WO2005/001570 A offenbart eine biokompatible, niedrigviskose, strahlungshärtbare Zusammensetzung zur Herstellung von Ohrpasstücken, welche ein Dimethylacrylat auf Basis von Bisphenon, Bisphenol A oder Bisphenol F, ein Urethanmethacrylat mit einer Funktionalität von n < 4 und einer Viskosität < 15 Pa s, ein

aliphatisches oder zykloaliphatisches Dimethacrylat, ein monofunktionelles Methacrylat, einen Photoinitiator und einen Inhibitor enthält.

Aufgabe der vorliegenden Erfindung ist es, eine Harzformulierung für die Produktion von Medizinprodukten, insbesondere von Ohrstücken mittels konventioneller PNP-Technik bzw. mittels generativer Herstellverfahren wie Stereolithographie (SLA) und Direct Light Processing (DLP), zur Verfügung zu stellen, die zum einen antimikrobielle Wirksamkeit aufweist und die mechanischen, insbesondere eine Dehnung von ≥ 10%, toxikologischen als auch die an die o.g. Verfahren gestellten Anforderungen erfüllt.

Es wurde nun überraschenderweise gefunden, dass ein niedrigviskoses Harzgemisch, das aus einem Urethan(meth)acrylat mit einer Funktionalität von n < 4 und einer Viskosität < 15 Pa s besteht, das außerdem ein oder mehrere voneinander verschiedene monomere oder oligomere Di(meth)acrylate auf Basis von Bisphenol A , weiterhin mindestens eine oder eine Kombination von antimikrobiell beziehungsweise bakteriostatisch wirkenden Komponenten enthält, für die Technik des PNP-Verfahrens oder der Stereolithographie beziehungsweise DLP eingesetzt werden kann und das bei der Härtung mittels Lasers vorgehärtete Formkörper ergibt, die sich durch eine hohe Grünfestigkeit auszeichnen.

Insbesondere sind durch die Zugabe eines in Gegenwart von Feuchtigkeit antimikrobiell wirkenden Glasfüllers im Konzentrationsbereich von 1-4 Gew.-%, niedrigviskose Harzformulierungen realisierbar, deren Biokompatibilität, deren chemisch-physikalischen Eigenschaften, deren Verhältnis von kritischer Energie zur Lasereindringtiefe den Anforderungen der o. g. Herstellverfahren in besonderer Weise gerecht werden können und mit denen sich zusätzlich besonders glatte Oberflächen der generierten Formkörper erzeugen lassen.

Darüber hinaus wurde weiterhin gefunden, dass durch geringen Zusatz eines anaeroben Inhibitors wie 2,2,6,6-Tetramethylpiperidin-1-yloxy (freies Radikal) die Lasereindringtiefe und die kritische Energie der Harzformulierungen vorteilhaft gesteuert werden können. Die durch Aushärtung erhaltenen Formkörper weisen neben guten mechanischen Eigenschaften exzellente Biokompatibilität und geringe Wasseraufnahmen auf, besitzen Bruchdehnungswerte ≥10 % und können nachbehandelt wie beispielsweise getumbled, geschliffen oder lackiert werden. Dabei erweist sich die glatte Oberfläche als vorteilhaft, da z.B. beim Tumbleprozess der Abschliff und dadurch die Tumblezeiten verringert werden. Dementsprechend werden somit die Prozesskosten geringer bei erhöhterer Formtreue und somit Passgenauigkeit der hergestellten zum Beispiel Ohrpassstücke.

Der Gegenstand der vorliegenden Erfindung ist dementsprechend eine niedrigviskose, strahlungshärtbare Harzformulierung für den Einsatz in der PNP-Technik bzw. der o. g. generativen Fertigungstechnologien, mittels derer antimikrobiell ausgestattete Objekte hergestellt werden können, enthaltend:
a) 20-75 Gew.-% eines oder mehrerer aliphatischer oder cycloaliphathischer monomerer/oligomerer Urethan(meth)acrylate mit einer Funktionalität von n < 4 , einer Viskosität < 15 Pa s und einem Molekülgewicht < 2000
b) 5-25 Gew.-% eines n-fach ethoxylierten Bisphenol-a-Dimethacrylats mit einem Ethoxylierungsgrad >10 oder einer Mischung nfach ethoxylierter Bisphenol-a-Dimethacrylate mit einem Ethoxylierungsgrad ≤ 30
c) 2,5-10 Gew.-% einer n-fach vernetzenden monomeren oder oligomeren Komponente, charakterisiert durch n ≥ 4 Meth- und/oder Acrylatfunktionen mit einer Viskosität ≤ 20 Pa s
d) 4-15 Gew.-% eines oder mehrer monofunktioneller (Meth)acrylate mit einer Viskosität < 3 Pa s
e) 1-4 Gew.-% eines antimikrobiell wirkenden Glasfüllers, bestehend aus 45±5 Gew.-% SiO₂, 25±5 Gew.-% Na₂O, 25±5 Gew.-% CaO und 5±5 Gew.-% P₂O₅ oder einer Kombination antimikrobieller Zusätze aus den Gruppen antimikrobiell wirksamer Glasfüller oder Silbernanopartikel
f) 1-4,5 Gew.-% eines oder einer Kombination mehrerer Photoinitiatoren, deren Absorption im Wellenlängenbereich des eingesetzten Laserstrahles bzw. der Bestrahlungsquelle liegt
g)0-0,5 Gew.-% eines oder einer Kombination von anaeroben Inhibitoren, auch in Verbindung mit denen, dem Stereolithographiefachmann bekannten aeroben Inhibitoren
h) 0-10 Gew.-% an Füllstoffen
i) 0-4 Gew.-% an Farbmitteln
j) 0,01-3 Gew.-% an üblichen Additiven wie UV-Stabilisatoren oder Verlaufsadditiven, wobei der Anteil der Komponenten a) bis j) zusammen 100 Gew.-% beträgt.

Die in den erfindungsgemäßen Formulierungen als Komponente (a) eingesetzten Urethan(meth)acrylate mit einer Funktionalität < 4 sind dem Fachmann bekannt und können in bekannter Weise hergestellt werden, indem man beispielsweise ein hydroxylterminiertes Polyurethan mit Methacrylsäure zum entsprechendem Urethanmethacrylat umsetzt, oder indem man ein isocyanatterminiertes Präpolymer mit Hydroxymethacrylaten umsetzt. Entsprechende Verfahren sind z.B. aus EP 0579503 bekannt. Urethan(meth)acrylate sind auch im Handel erhältlich und werden beispielsweise unter der Bezeichnung PC-Cure® von der Firma Piccadilly Chemicals, unter der Produktbezeichnung CN 1963 von der Firma Sartomer, unter der Bezeichnung Photomer von der Firma Cognis, unter der Bezeichnung Ebecryl von der Firma UCB und unter der Bezeichnung Genomer® von der Firma Rahn vertrieben.
Als Urethan(meth)acrylate werden solche eingesetzt, die n < 4 funktionalisiert sind, Viskositäten <15 Pa s besitzen, ein Molekülgewicht < 2000 haben, und aus aliphatischen Edukten hergestellt worden sind. Insbesondere findet das aus HEMA und TMDI erhaltene Isomerengemisch 7,7,9-(beziehungsweise 7,9,9-)Trimethyl-4,13-dioxo-3,14-dioxa-5,12-diazahexadecan-1,16-dioldimethacrylat Anwendung.
Als Verbindungen der Komponente (b) eignen sich beispielsweise Dimethacrylate des (n)-alkoxylierten Bisphenol A wie Bisphenol-A-ethoxylat(2)dimethacrylat, Bisphenol-A-ethoxylat(4)dimethacrylat, Bisphenol-A-propoxylat(2)dimethacrylat, Bisphenol-A-propoxylat(4)dimethacrylat sowie Dimethacrylate des (n)-alkoxylierten Bisphenol F wie Bisphenol-F-ethoxylat(2)dimethacrylat und Bisphenol-F-ethoxylat(4)dimethacrylat , Bisphenol-F-propoxylat(2)dimethacrylat, Bisphenol-F-propoxylat(4)dimethacrylat und Mischungen dieser.
Vorzugsweise verwendet man monomere oder oligomere Dimethacrylate auf Basis von Bisphenol A, insbesondere das Bisphenol-A-ethoxylat(10)dimethacrylat und das Bisphenol-A-ethoxylat(30)dimethacrylat.
Als Verbindungen der Komponente (b) können beispielsweise weiterhin eingesetzt werden: 1,3-Butandioldimethacrylat, 1,6-Hexandioldimethacrylat, 1,3-Butylenglykoldimethacrylat, Diethylenglykoldimethacrylat, Ethylenglykoldimethacrylat, Neopentyldimethacrylat, Polyethylenglykoldimethacrylat, Triethylenglykoldimethacrylat und Tetraethylenglykoldimethacrylat und bevorzugt 1,4-Butandioldimethacrylat. Solche Produkte sind im Handel erhältlich, beispielsweise von der Firma Sartomer.
Als Verbindungen der Komponente (c) können beispielsweise eingesetzt werden:
Di-Trimethylolpropantetra(meth)acrylat,
Dipentaerythritol-penta(meth)acrylat, nfach ethoxyliertes Dipentaerythritolpenta(meth)acrylat,
Pentaerythritoltetra(meth)acrylat. Solche Produkte sind im Handel erhältlich, beispielsweise von der Fa. Sartomer.
Als Verbindungen der Komponente (d) können beispielsweise eingesetzt werden: 2(2-ethoxyethoxy)ethyl(meth)acrylat, Phenoxyethyl(meth)acrylat, C12-C18alkyl(meth)acrylate, Caprolacton(meth)acrylat, Isobornyl(meth)acrylat, Isodecyl(meth)acrylat, Lauryl(meth)acrylat, Polypropylenglykol(meth)acrylat, Tetrahydrofurfuryl(meth)acrylat.
Als Komponente (e) kommen in Anwesenheit von Feuchtigkeit antimikrobiell wirkende Glasfüller, bestehend aus 45±5 Gew.-% SiO₂, 25±5 Gew.-% Na₂O, 25±5 Gew.-% CaO und 5±5 Gew.-% P₂O₅, mit einer Partikelgrösse <100 µm, wie sie bspw. Von der Fa. Schott vertrieben werden, einzeln oder in Kombination mit Ag-Nanopartikeln, wie sie u. a. von der Fa. Ciba oder Fa. AgPure im Handel erhältlich sind. Die Wirkungsweisen der bakterostatischen Effekte der beschriebenen Zusätze sind vielfältig (pH-Wertsteuerung, Veränderung des osmotischen Drucks an der Zellmembran, Kaliumioneaustausch und irreversible Reaktionen mit thiohaltigen Proteinen). Diese sind dem Fachmann bekannt und können in der einschlägigen Literatur nachverfolgt werden.
Als Komponente (f) können als Photoinitiatoren alle Typen eingesetzt werden, die bei der entsprechenden Bestrahlung freie Radikale bilden. Dabei sind bekannte Photoinitiatoren Verbindungen der Benzoine, Benzoinether, wie Benzoin, Benzoinmethylether, Benzoinethylether und Benzoinisopropylether, Benzoinphenylether und Benzoinacetat, Acetophenone, wie Acetophenon, 2,2-Dimethoxyacetophenon, und 1,1-Dichloracetophenon, Benzil, Benzilketale, wie Benzildimethylketal und Benzildiethylketal, Anthrachinone, wie 2-Methylanthrachinon, 2-Ethylanthrachinon, 2-tert.-Butylanthrachinon, 1-Chloranthrachinon und 2-Amylanthrachinon, Triphenylphosphin, Benzoylphosphinoxide, wie beispielsweise 2,4,6-Trimethylbenzoyldiphenylphosphinoxid (Luzirin TPO) und Bis(2,4,6-trimethylbenzoylphenyl)-phosphinoxid, Benzophenone, wie Benzophenon und 4,4'-Bis-(N,N'-dimethylamino)-benzophenon, Thioxanthone und Xanthone, Acridinderivate, Phenazinderivate, Quinoxalinderivate oder 1-Phenyl-1,2-propandion-2-O-benzoyloxim, 1-Aminophenylketone oder 1-Hydroxyphenylketone, wie 1-Hydroxycyclohexylphenylketon, Phenyl-(1-hydroxyisopropyl)-keton und 4-Isopropylphenyl-(1-hydroxyisopropyl)-keton.
Besonders bevorzugte Verbindungen, die in Kombination mit einem Nd:YVO₄-Festkörperlaser eingesetzt werden, sind Bis(2,4,6-trimethylbenzoyl)-phenylphosphinoxid, 2,4,6-Trimethylbenzoyldiphenylphosphinoxid, 2-Hydroxy-2-methylpropiophenon, Hydroxycyclohexylphenylketon und Mischungen dieser Photoinitiatoren.
In den erfindungsgemäßen Gemischen (g) können als anaerobe Inhibitoren bzw. Stabilisatoren das 2,2,6,6-Tetramethylpiperidin-1-yloxy (freies Radikal), das Phenothiazin (PTZ), das 2,2-Diphenyl-1-picrylhydrazyl-radikal (DPPH) oder eine Kombination der Inhibitoren zur Einstellung der Lasereindringtiefe und der kritischen Energie zugesetzt werden.
Den erfindungsgemäßen Gemischen können, falls erforderlich, die dem Fachmann bekannten Füllstoffe und Additive zugesetzt werden, beispielsweise Verlaufsmittel, UV-Stabilisatoren, Benetzungsmittel, Füllstoffe, Farbstoffe und Pigmente. Im Sinne der Erfindung besonders geeignete Farbstoffe sind Anthrachinon-Farbstoff-Zubereitungen, wie sie z. B. von der Firma Bayer unter dem Namen Macrolex® verkauft werden.
Aus den Beispielen 1-6 kann man die Formulierungen erfindungsgemäßer, transluzenter Harzmischungen in den Varianten mit und ohne zugefügtem antimikrobiellen Glasfüller entnehmen, wie sie zum Einsatz von z.B. zur Herstellung von Otoplastiken kommen könnten.

**Beispiel 1:** eines farblos, transluzenten Stereolithographieharzes mit antimikrobiellen Eigenschaften:
- 65,6 Gew.-%: 7,7,9-(bzw.7,9,9-)Trimethyl-4,13-dioxo-3,14-dioxa-5,12diaza-hexadecan-1,16-dioldimethacrylat
- 14,672 Gew.-%: Bisphenol-A-ethoxylat(30)dimethacrylat
- 4,8 Gew.-%: Dipentaerythritolpenta(meth)acrylat
- 4,6 Gew.-%: 1,6-Hexandioldimethacrylat
- 4,6 Gew.-%: Laurylmethacrylat methacrylat
- 3 Gew.-%: 1-Hydroxycyclohexylphenylketon
- 1,5 Gew.-%: Glasfüller, bestehend aus 45±5 Gew.-% SiO₂, 25±5 Gew.-% Na₂O, 25±5 Gew.-% CaO und 5±5 Gew.-% P₂O₅;
- 1 Gew.-%: 2-Hydroxy-2methyl-1phenylpropanon
- 0,2 Gew.-%: 2,4,6-Trimethylbenzoyldiphenylphosph inoxid
- 0,025 Gew.-%: UV-Stabilisator
- 0,003 Gew.-%: 2,2,6,6-Tetramethylpiperidin-1-yloxy (freies Radikal)

**Beispiel 2:** eines farblos, transluzenten Stereolithographieharzes ohne antimikrobiellen Zusatz:
- 66,62 Gew.-%: 7,7,9-(bzw.7,9,9-)Trimethyl-4,13-dioxo-3,14-dioxa-5,12diazahexadecan-1,16-dioldimethacrylat
- 14,92 Gew.-%: Bisphenol-A-ethoxylat(30)dimethacryla t
- 4,88 Gew.-%: Dipentaerythritolpenta(meth)acrylat
- 4,68 Gew.-%: 1,6-Hexandioldimethacrylat
- 4,68 Gew.-%: laurylmethacrylat
- 3 Gew.-%: 1-Hydroxycyclohexylphenylketon
- 1 Gew.-%: 2-Hydroxy-2methyl-1phenylpropanon
- 0,2 Gew.-%: 2,4,6-Trimethylbenzoyldiphenylphosphinoxid
- 0,025 Gew.-%: UV-Stabilisator
- 0,003 Gew.-%: 2,2,6,6-Tetramethylpiperidin-1-yloxy (freies Radikal)

**Beispiel 3:** eines roten, transluzenten Stereolithographieharzes mit antimikrobiellen Eigenschaften:
- 67,65 Gew.-%: 7,7,9-(bzw.7,9,9-)Trimethyl-4,13-dioxo-3,14-dioxa-5,12diaza-hexadecan-1,16-dioldimethacrylat
- 15 Gew.-%: Bisphenol-A-ethoxylat(30)dimethacrylat
- 4,9 Gew.-%: Dipentaerythritol-penta(meth)-acrylat
- 4,7 Gew.-%: 1,6-Hexandioldimethacrylat
- 4,7 Gew.-%: Laurylmethacrylat
- 1,5 Gew.-%: 2,4,6-Trimethylbenzoyldiphenylphosphinoxid
- 1,5 Gew.-%: Glasfüller, bestehend aus 45±5 Gew.-% SiO₂, 25±5 Gew.-% Na₂O, 25±5 Gew.-% CaO und 5±5 Gew.-% P₂O₅
- 0,025 Gew.-%: UV-Stabilisator
- 0,012 Gew.-%: Azofarbstoff Rot H
- 0,003 Gew.-%: 2,2,6,6-Tetramethylpiperidin-1-yloxy (freies Radikal)

**Beispiel 4:** eines roten, transluzenten Stereolithographieharzes ohne antimikrobiellen Zusatz:
- 69,15 Gew.-%: 7,7,9-(bzw.7,9,9-)Trimethyl-4,13-dioxo-3,14-dioxa-5,12diaza-hexadecan-1,16-dioldimethacrylat
- 15 Gew.-%: Bisphenol-A-ethoxylat(30)dimethacrylat
- 4,9 Gew.-%: Dipentaerythritol-penta(meth)-acrylat
- 4,7 Gew.-%: 1,6-Hexandioldimethacrylat
- 4,7 Gew.-%: Laurylmethacrylat
- 1,5 Gew.-%: 2,4,6-Trimethylbenzoyldiphenylphosphinoxid
- 0,025 Gew.-%: UV-Stabilisator
- 0,012 Gew.-%: Azofarbstoff Rot H
- 0,003 Gew.-%: 2,2,6,6-Tetramethylpiperidin-1-yloxy (freies Radikal)

**Beispiel 5:** eines blauen, transluzenten Stereolithographieharzes mit antimikrobiellen Eigenschaften:
- 67,65 Gew.-%: 7,7,9-(bzw.7,9,9-)Trimethyl-4,13-dioxo-3,14-dioxa-5,12diaza-hexadecan-1,16-dioldimethacrylat
- 15 Gew.-%: Bisphenol-A-ethoxylat(30)dimethacrylat
- 4,9 Gew.-%: Dipentaerythritol-penta(meth)-acrylat
- 4,7 Gew.-%: 1,6-Hexandioldimethacrylat
- 4,7 Gew.-%: Laurylmethacrylat
- 1,5 Gew.-%: 2,4,6-Trimethylbenzoyldiphenylphosphinoxid
- 1,5 Gew.-%: Glasfüller, bestehend aus 45±5 Gew.-% SiO₂, 25±5 Gew.-% Na₂O, 25±5 Gew.-% CaO und 5±5 Gew.-% P₂O₅
- 0,025 Gew.-%: UV-Stabilisator
- 0,03 Gew.-%: Anthrachinon-Farbstoff-Zubereitung (inkl.C.I. Solvent Blue97)
- 0,003 Gew.-%: 2,2,6,6-Tetramethylpiperidin-1-yloxy (freies Radikal)

**Beispiel 6:** eines blauen, transluzenten Stereolithographieharzes ohne antimikrobiellen Zusatz:
- 68,55 Gew.-%: 7,7,9-(bzw.7,9,9-)Trimethyl-4,13-dioxo-3,14-dioxa-5,12diaza-hexadecan-1,16-dioldimethacrylat
- 15,3 Gew.-%: Bisphenol-A-ethoxylat(30)dimethacrylat
- 5 Gew.-%: Dipentaerythritol-penta(meth)-acrylat
- 4,8 Gew.-%: 1,6-Hexandioldimethacrylat
- 4,8 Gew.-%: Laurylmethacrylat
- 1,5 Gew.-%: 2,4,6-Trimethylbenzoyldiphenylphosphinoxid
- 0,025 Gew.-%: UV-Stabilisator
- 0,03 Gew.-%: Anthrachinon-Farbstoff-Zubereitung (inkl.C.I. Solvent Blue97)
- 0,003 Gew.-%: 2,2,6,6-Tetramethylpiperidin-1-yloxy (freies Radikal)

Die chemisch-physikalischen Daten der Beispielformulierungen 1-6 sind in Tab.1 aufgeführt.

**Tab.1: Parameter der Stereolithographieharzformulierungen aus Bsp.1 bis Bsp. 6**

| **Eigenschaft** | **Bsp.1** | **Bsp.2** | **Bsp. 3** | **Bsp. 4** | **Bsp. 5** | **Bsp.6** |
|---|---|---|---|---|---|---|
| Viskosität bei 23°C, mPa s | 1000 | 1060 | 1240 | 1120 | 1180 | 1070 |
| E-Modul des ausgehärteten Formkörpers, N mm⁻² | 1739 | 1739 | 2002 | 1963 | 2033 | 1978 |
| Biegefestigkeit des ausgehärteten Formkörpers, N mm⁻² | 97 | 98 | 112 | 110 | 112 | 113 |
| Bruchdehnung des ausgehärteten Formkörpers, % | 11 | 11 | 11 | 11 | 10 | 10 |
| Ec, mJ cm⁻² | 21 | 20,9 | 14,5 | 14 | 14,4 | 14,4 |
| Dp, mils | 6,6 | 5,4 | 6,3 | 5,1 | 5,7 | 5,6 |

Die für die Technik der Stereolithographie relevanten Parameter der o. g. Harze sind in Tab.1 aufgeführt. Sämtliche Viskositätsmessungen wurden bei 23°C mit einem CVO 120-Rheometer der Fa. Bohlin Instruments durchgeführt. Die Bestimmung der Biegefestigkeiten, E-Module und Dehnungen wurde in Anlehnung an die EN ISO 178 (1996) mit einer Zwick 1-Testmaschine der Fa. Zwick durchgeführt. Zur Ermittlung der Ec und Dp-Werte mittels der o. g. Windowpane-Methode wurden jeweils die Mittelwerte aus 10 Windowpane-Probekörpern ermittelt. Die Probekörper wurden mit einer Viper si² SLA Anlage (Fa. 3D Systems), ausgerüstet mit einem Nd:YVO₄ Festkörperlaser, hergestellt. Die Grünlinge wurden mit der Stroboskop-Belichtungseinheit Sonolux PR der Fa. Innovation Meditech 2x4800 Blitze nachgehärtet.
Der Tab.1 kann man entnehmen, dass durch die Zugabe des antimikrobiell wirkenden Glasfüllers im Rahmen der Messgenauigkeiten vergleichbare Ergebnisse zu den Systemen ohne Glasfüller erreicht werden. Im direkten Vergleich zu den ungefüllten Systemen erhält man jedoch bei den gefüllten Systemen Formkörper mit wesentlich homogeneren Oberflächen. Ferner lässt sich durch die Glasfüllerzugabe ein leichter Anstieg der Lasereindringtiefe beobachten. Dieser Effekt kann im Hinblick auf höherer Schichtstärken und somit verkürzte Bauzeiten genutzt werden. Darüber hinaus ergibt sich aus Tab.1, dass insbesondere durch die Zugabe des antimikrobiell wirkenden Füllers Harzformulierungen erhalten werden, die denen dem Stand der Technik (siehe Tab.2) in der Gesamtheit ihrer mechanischen Werte überlegen sind.

**Tab.2: Mechanische Werte kommerziell erhältlicher Produkte für die Herstellung von Ohrstücken**

| **Material** | **E-Modul, N mm⁻²** | **Biegefestigkeit, N** | **Bruchdehnung.** |
|---|---|---|---|
| | | **mm⁻²** | **%** |
| Fotoplast S/IO blau transparent, Lot. 201504 | 1513 | 81 | 10 |
| Fotoplast S/IO rot transparent. Lot. 301531 | 1527 | 84 | 13 |
| Fotoplast S/IO farblos transparent, Lot. 203523 | 1602 | 88 | 11 |
| Fotoplast S/IO gelblich,Lot. 301515 | 1586 | 80 | 13 |

Neben transluzenten
Stereolithographieharzformulierungen kommen auch opak eingestellte Rezepturen häufig z. B. für den Bau von Hörgeräteschalen zum Einsatz.

**Beispiel 7:** eines beige, opaken Stereolithographieharzes mit antimikrobiellen Glasfüller:
- 66,84 Gew.-%: 7,7,9-(bzw.7,9,9-)Trimethyl-4,13-dioxo-3,14-dioxa-5,12diaza-hexadecan-1,16-dioldimethacrylat
- 14,92 Gew.-%: Bisphenol-A-ethoxylat(30)dimethacrylat
- 4,88 Gew.-%: Dipentaerythritolpenta(meth)acrylat
- 4,68 Gew.-%: 1,6-Hexandioldimethacrylat
- 4,68 Gew.-%: Laurylmethacrylat
- 2,0 Gew.-%: Glasfüller, bestehend aus 45±5 Gew.-% SiO₂, 25±5 Gew.-% Na₂O, 25±5 Gew.-% CaO und 5±5 Gew.-% P₂O₅
- 1,5 Gew.-%: Phenylbis(2,4,6-trimethylbenzoyl)-phosphinoxid
- 0,025 Gew.-%: UV-Stabilisator
- 0,5 Gew.-%: Eisenoxidpigmente
- 0,003 Gew.-%: 2,2,6,6-Tetramethylpiperidin-1-yloxy (freies Radikal)

**Beispiel 8:** eines beige, opaken Stereolithographieharzes ohne antimikrobiellen Glasfüller:
- 68,84 Gew.-%: 7,7,9-(bzw.7,9,9-)Trimethyl-4,13-dioxo-3,14-dioxa-5,12diaza-hexadecan-1,16-dioldimethacrylat
- 14,92 Gew.-%: Bisphenol-A-ethoxylat(30)dimethacrylat
- 4,88 Gew.-%: Dipentaerythritol-penta(meth)-acrylat
- 4,68 Gew.-%: 1,6-Hexandioldimethacrylat
- 4,68 Gew.-%: Laurylmethacrylat
- 1,5 Gew.-%: Phenylbis(2,4,6-trimethylbenzoyl)-phosphinoxid
- 0,025 Gew.-%: UV-Stabilisator
- 0,5 Gew.-%: Eisenoxidpigmente
- 0,003 Gew.-%: 2,2,6,6-Tetramethylpiperidin-1-yloxy (freies Radikal)

Bevorzugt in opaken Formulierungen lassen sich Silberpartikel als antimikrobiell wirkender Zusatz im Konzentrationsbereich von 0,1 bis 1 Gew.-% einarbeiten.

**Beispiel 9:** eines beige, opaken Stereolithographieharzes mit antimikrobiell wirkenden Silberpartikeln:
- 68,74 Gew.-%: 7,7,9-(bzw.7,9,9-)Trimethyl-4,13-dioxo-3,14-dioxa-5,12diaza-hexadecan-1,16-dioldimethacrylat
- 14,92 Gew.-%: Bisphenol-A-ethoxylat(30)dimethacrylat
- 4,88 Gew.-%: Dipentaerythritolpenta(meth)acrylat
- 4,68 Gew.-%: 1,6-Hexandioldimethacrylat
- 4,68 Gew.-%: Laurylmethacrylat
- 1,5 Gew.-%: Phenylbis(2,4,6-trimethylbenzoyl)-phosphinoxid
- 0,025 Gew.-%: UV-Stabilisator
- 0,5 Gew.-%: Eisenoxidpigmente
- 0,1 Gew.-%: Nanosilber (Partikelgröße < 30 nm)
- 0,003 Gew.-%: 2,2,6,6-Tetramethylpiperidin-1-yloxy (freies Radikal)

**Beispiel 10:** eines beige, opaken Stereolithographieharzes mit antimikrobiell wirkenden Silberpartikeln:
- 68,54 Gew.-%: 7,7,9-(bzw.7,9,9-)Trimethyl-4,13-dioxo-3,14-dioxa-5,12diaza-hexadecan-1,16-dioldimethacrylat
- 14,92 Gew.-%: Bisphenol-A-ethoxylat(30)dimethacrylat
- 4,88 Gew.-%: Dipentaerythritol-penta(meth)-acrylat
- 4,68 Gew.-%: 1,6-Hexandioldimethacrylat
- 4,68 Gew.-%: Laurylmethacrylat
- 1,5 Gew.-%: Phenylbis(2,4,6-trimethylbenzoyl)-phosphinoxid
- 0,025 Gew.-%: UV-Stabilisator
- 0,5 Gew.-%: Eisenoxidpigmente
- 0,3 Gew.-%: Nanosilber (Partikelgröße < 30 nm)
- 0,003 Gew.-%: 2,2,6,6-Tetramethylpiperidin-1-yloxy (freies Radikal)

**Beispiel 11:** eines beige, opaken Stereolithographieharzes mit antimikrobiell wirkenden Silberpartikeln:
- 67,84 Gew.-%: 7,7,9-(bzw.7,9,9-)Trimethyl-4,13-dioxo-3,14-dioxa-5,12diaza-hexadecan-1,16-dioldimethacrylat
- 14,92 Gew.-%: Bisphenol-A-ethoxylat(30)dimethacrylat
- 4,88 Gew.-%: Dipentaerythritol-penta(meth)-acrylat
- 4,68 Gew.-%: 1,6-Hexandioldimethacrylat
- 4,68 Gew.-%: Laurylmethacrylat
- 1,5 Gew.-%: Phenylbis(2,4,6-trimethylbenzoyl)-phosphinoxid
- 0,025 Gew.-%: UV-Stabilisator
- 0,5 Gew.-%: Eisenoxidpigmente
- 1 Gew.-%: Nanosilber (Partikelgröße < 30 nm)
- 0,003 Gew.-%: 2,2,6,6-Tetramethylpiperidin-1-yloxy (freies Radikal)

**Tab.3: Parameter der beige opaken Stereolithographieharzformulierungen aus Bsp.7 bis Bsp. 11**

| **Eigenschaft** | **Bsp.7** | **Bsp.8** | **Bsp.9** | **Bsp10** | **Bsp.11** |
|---|---|---|---|---|---|
| Viskosität bei 23°C, mPa s | 1270 | 1320 | 1200 | 1220 | 1220 |
| E-Modul des ausgehärteten Formkörpers, N mm⁻² | 1978 | 1998 | 1932 | 1857 | 1852 |
| Biegefestigkeit des ausgehärteten Formkörpers, N mm⁻² | 113 | 99 | 103 | 106 | 103 |
| Bruchdehnung des ausgehärteten Formkörpers, % | 13 | 7 | 10 | 11 | 10 |
| Ec, mJ cm⁻² | 9,4 | 15,9 | 13 | 11 | 11,7 |
| Dp, mils | 3,8 | 2,0 | 3,2 | 2,9 | 2,6 |

Aus den in Tab. 3 aufgeführten Werten ist ersichtlich, dass die Bsp. 7-11 ebenfalls zu Stereolithographieharzen führen, die zur Herstellung von Hörgeräteschalen vorteilhaft eingesetzt werden können. Ferner wurde beobachtet, dass die Bruchdehnungswerte für o.g. Harzmischungen sich um 3% erhöhen, wenn die Probekörper mittels des Stereolithographieprozesses hergestellt werden. In einer besonderen Ausführungsform enthalten die Harzmischungen neben einem antimikrobiell wirkenden Glasfüller auch Silberpartikel (Bsp.12-14).

**Beispiel 12:** eines beige, opaken Stereolithographieharzes mit antimikrobiell wirkenden Silberpartikeln und Glasfüller:
- 67,74 Gew.-%: 7,7,9-(bzw.7,9,9-)Trimethyl-4,13-dioxo-3,14-dioxa-5,12diaza-hexadecan-1,16-dioldimethacrylat
- 14,42 Gew.-%: Bisphenol-A-ethoxylat(30)dimethacrylat
- 4,88 Gew.-%: Dipentaerythritol-penta(meth)-acrylat
- 4,68 Gew.-%: 1,6-Hexandioldimethacrylat
- 4,68 Gew.-%: Laurylmethacrylat
- 1,5 Gew.-%: Phenylbis(2,4,6-trimethylbenzoyl)-phosphinoxid
- 1,5 Gew.-%: Glasfüller, bestehend aus 45±5 Gew.-% SiO₂, 25±5 Gew.-% Na₂O, 25±5 Gew.-% CaO und 5±5 Gew.-% P₂O₅
- 0,025 Gew.-%: UV-Stabilisator
- 0,5 Gew.-%: Eisenoxidpigmente
- 0,1 Gew.-%: Nanosilber (Partikelgröße < 30 nm)
- 0,003 Gew.-%: 2,2,6,6-Tetramethylpiperidin-1-yloxy (freies Radikal)

**Beispiel 13:** eines beige, opaken Stereolithographieharzes mit antimikrobiell wirkenden Silberpartikeln und Glasfüller:
- 67,54 Gew.-%: 7,7,9-(bzw.7,9,9-)Trimethyl-4,13-dioxo-3,14-dioxa-5,12diaza-hexadecan-1,16-dioldimethacrylat
- 14,42 Gew.-%: Bisphenol-A-ethoxylat (30)dimethacrylat
- 4,88 Gew.-%: Dipentaerythritol-penta(meth)-acrylat
- 4,68 Gew.-%: 1,6-Hexandioldimethacrylat
- 4,68 Gew.-%: Laurylmethacrylat
- 1,5 Gew.-%: Phenylbis(2,4,6-trimethylbenzoyl)-phosphinoxid 1,5 Gew.-% Glasfüller, bestehend aus 45±5 Gew.-% SiO₂, 25±5 Gew.-% Na₂O, 25±5 Gew.-% CaO und 5±5 Gew.-% P₂O₅
- 0,025 Gew.-%: UV-Stabilisator
- 0,5 Gew.-%: Eisenoxidpigmente
- 0,3 Gew.-%: Nanosilber (Partikelgröße < 30 nm)
- 0,003 Gew.-%: 2,2,6,6-Tetramethylpiperidin-1-yloxy (freies Radikal)

**Beispiel 14:** eines beige, opaken Stereolithographieharzes mit antimikrobiell wirkenden Silberpartikeln und Glasfüller:
- 66,84 Gew.-%: 7,7,9-(bzw.7,9,9-)Trimethyl-4,13-dioxo-3,14-dioxa-5,12diaza-hexadecan-1,16-dioldimethacrylat
- 14,42 Gew.-%: Bisphenol-A-ethoxylat(30)dimethacrylat
- 4,88 Gew.-%: Dipentaerythritol-penta(meth)-acrylat
- 4,68 Gew.-%: 1,6-Hexandioldimethacrylat
- 4,68 Gew.-%: Laurylmethacrylat
- 1,5 Gew.-%: Phenylbis(2,4,6-trimethylbenzoyl)phosphinoxid
- 1,5 Gew.-%: Glasfüller, bestehend aus 45±5 Gew.-% SiO₂, 25±5 Gew.-% Na₂O, 25±5 Gew.-% CaO und 5±5 Gew.-% P₂O₅
- 0,025 Gew.-%: UV-Stabilisator
- 0,5 Gew.-%: Eisenoxidpigmente
- 1 Gew.-%: Nanosilber (Partikelgröße < 30 nm)
- 0,003 Gew.-%: 2,2,6,6-Tetramethylpiperidin-1-yloxy (freies Radikal)

Die chemisch-physikalischen Parameter der Beispielformulierungen sind in Tab. 4 zusammengestellt.

**Tab.4: Parameter der beige opaken Stereolithographieharzformulierungen aus Bsp.12 Bis Bsp. 14, die sowohl Silberpartikel als auch Glasfüller mit antimikrobieller Wirkung enthalten:**

| **Eigenschaft** | **Bsp.12** | **Bsp.13** | **Bsp.14** |
|---|---|---|---|
| Viskosität bei 23°C, mPa s | 1470 | 135 0 | 1370 |
| E-Modul des ausgehärteten Formkörpers, Nmm⁻² | 1956 | 189 7 | 1854 |
| Biegefestigkeit des ausgehärteten Formkörpers, N mm⁻² | 105 | 100 | 96 |
| Bruchdehnung des ausgehärteten Formkörpers, % | 10 | 9 | 9 |
| Ec, mJ cm⁻² | 9,2 | 10,3 | 9,8 |
| Dp, mils | 3,1 | 2,9 | 2,6 |

Aus den in Tab. 4 aufgeführten Werten wird deutlich, dass die Bsp. 12-14 ebenfalls zu Stereolithographieharzen führen, die zur Herstellung von Hörgeräteschalen vorteilhaft eingesetzt werden können. Ferner wurde auch hier beobachtet, dass die Bruchdehnungswerte für o. g. Harzmischungen sich um 3% erhöhen, wenn die Probekörper mittels des Stereolithographieprozesses hergestellt werden. Es werden somit niedrigviskose Harzmischungen bereitgestellt, mittels derer Ohrpassstücke generativ hergestellt werden können, die sich in der Gesamtheit ihrer mechanischen Eigenschaften durch hohe Werte auszeichnen und gleichzeitig eine antimikrobiell ausgestattete, zum Stand der Technik wesentlich glattere Oberfläche aufweisen. Dementsprechend müssen die Ohrpassstücke nicht durch aufwändige Lackierungprozesse vor der Besiedelung mit Biofilmen geschützt werden.

## Patentansprüche

1. Biokompatible, niedrigviskose, strahlungshärtbare Formulierung, zur Herstellung von antimikrobiell ausgestatteten Medizinprodukten, insbesondere Ohrpassstücken, mittels PNP bzw. generativer Herstellverfahren, enthaltend:
a) 20-75 Gew.-% eines oder mehrerer aliphatischer oder cycloaliphathischer monomerer/oligomerer Urethan(meth)acrylate mit einer Funktionalität von n < 4, einer Viskosität < 15 Pa s und einem Molekülgewicht < 2000
b) 5-25 Gew.-% eines n-fach ethoxylierten Bisphenol-a-Dimethacrylats mit einem Ethoxylierungsgrad >10 oder einer Mischung n-fach ethoxylierter Bisphenol-a-Dimethacrylate mit einem Ethoxylierungsgrad ≤ 30
c) 2,5-10 Gew.-% einer n-fach vernetzenden monomeren oder oligomeren Komponente, charakterisiert durch n ≥ 4 Meth- und/oder Acrylatfunktionen mit einer Viskosität ≤ 20 Pa s
d) 4-15 Gew.-% eines oder mehrer monofunktioneller (Meth)acrylate mit einer Viskosität < 3 Pa s
e) 1-4Gew.-% eines antimikrobiell wirkenden Glasfüllers, bestehend aus 45±5 Gew.-% SiO₂, 25±5 Gew.-% Na₂O, 25±5 Gew.-% CaO und 5±5 Gew.-% P₂O₅, oder einer Kombination antimikrobieller Zusätze aus den Gruppen antimikrobiell wirksamer Glasfüller oder Silbernanopartikel
f) 1-4,5 Gew.-% eines oder einer Kombination mehrerer Photoinitiatoren, deren Absorption im Wellenlängenbereich des eingesetzten Laserstrahles bzw. der Bestrahlungsquelle liegt
g) 0-0,5 Gew.-% eines oder einer Kombination von anaeroben Inhibitoren, auch in Verbindung mit denen, dem Stereolithographiefachmann bekannten aeroben Inhibitoren
h) 0-10 Gew.-% an Füllstoffen
i) 0-4 Gew.-% an Farbmitteln
j) 0,01-3 Gew.-% an üblichen Additiven wie UV-Stabilisatoren oder Verlaufsadditiven, wobei der Anteil der Komponenten a) bis j) zusammen 100 Gew.-% beträgt.

2. Formulierung nach Anspruch 1, enthaltend
e) 1-2Gew.-% eines antimikrobiell wirkenden Glasfüllers bestehend aus 45 Gew.-% SiO₂, 25 Gew.-% Na₂O, 25 Gew.-% CaO und 5 Gew.-% P₂O₅
f) 0,01-1 Gew.-% an Silberpartikeln mit einem Partikeldurchmesser < 30nm.

3. Formulierung nach Anspruch 1 oder 2, enthaltend
a) 20-75 Gew.-% eines oder mehrerer aliphatischer oder cycloaliphathischer monomerer/oligomerer Urethan(meth)acrylate mit einer Funktionalität von n < 4, einer Viskosität < 15 Pa s und einem Molekülgewicht < 2000
b) 5-25 Gew.-% eines n-fach ethoxylierten Bisphenol-a-Dimethacrylats mit einem Ethoxylierungsgrad >10 oder einer Mischung n-fach ethoxylierter Bisphenol-a-Dimethacrylate mit einem Ethoxylierungsgrad ≤ 30
c) 2,5-10 Gew.-% einer n-fach vernetzenden monomeren oder oligomeren Komponente, charakterisiert durch n ≥ 4 Meth- und/oder Acrylatfunktionen mit einer Viskosität ≤ 20 Pa s
d) 4-15 Gew.-% eines oder mehrer monofunktioneller (Meth)acrylate mit einer Viskosität < 3 Pa s
e) 1-2Gew.-% eines antimikrobiell wirkenden Glasfüllers bestehend aus 45 Gew.-% SiO₂, 25 Gew.-% Na₂O, 25 Gew.-% CaO und 5 Gew.-% P₂O₅
f) 0,01-1 Gew.-% an Silberpartikeln mit einem Partikeldurchmesser < 30nm
g) 1-4,5 Gew.-% eines oder einer Kombination mehrerer Photoinitiatoren, deren Absorption im Wellenlängenbereich des eingesetzten Laserstrahles bzw. der Bestrahlungsquelle liegt
h) 0-0,5 Gew.-% eines oder einer Kombination von anaeroben Inhibitoren, auch in Verbindung mit denen, dem Stereolithographiefachmann bekannten aeroben Inhibitoren
i) 0-10 Gew.-% an Füllstoffen
j) 0-4 Gew.-% an Farbmitteln
k) 0,01-3 Gew.-% an üblichen Additiven wie UV-Stabilisatoren oder Verlaufsadditiven, wobei der Anteil der Komponenten a) bis j) zusammen 100 Gew.-% beträgt.

## Claims

1. A biocompatible, low-viscosity, radiation-curable formulation for the preparation of medical products with antimicrobial properties, in particular ear pieces, by means of PNP or generative preparation methods, containing:
a) 20 - 75 % by weight of one or a plurality of aliphatic or cycloaliphatic monomeric/oligomeric urethane (meth)acrylates having a functionality n < 4, a viscosity < 15 Pas, and a molecular weight < 2000,
b) 5 - 25 % by weight of an n-fold ethoxylated bisphenol A dimethacrylate having an ethoxylation degree > 10 or a mixture of n-fold ethoxylated bisphenol A dimethacrylates having an ethoxylation degree ≤ 30,
c) 2.5 - 10 % by weight of an n-fold cross-linking monomeric or oligomeric component, **characterized by** n ≥ 4 methacrylate and/or acrylate functions having a viscosity ≤ 20 Pas,
d) 4 - 15 % by weight of one or a plurality of monofunctional (meth)acrylates having a viscosity < 3 Pa s,
e) 1 - 4 % by weight of an antimicrobially acting glass filler, comprising 45 ± 5 % by weight of SiO₂, 25 ± 5 % by weight of Na₂O, 25 ± 5 % by weight of CaO, and 5 ± 5 % by weight of P₂O₅, or a combination of antimicrobial additives from the groups of antimicrobially acting glass fillers or silver nano particles,
f) 1 - 4.5 % by weight of one or a combination of a plurality of photoinitiators, the absorption of which is in the wavelength range of the laser beam or of the radiation source used,
g) 0 - 0.5 % by weight of one or a combination of anaerobic inhibitors, also in conjunction with aerobic inhibitors known to the man skilled in the art of stereo lithography,
h) 0 - 10 % by weight of fillers,
i) 0 - 4 % by weight of colorants,
j) 0.01 - 3 % by weight of conventional additives such as UV stabilizers or flow additives, wherein the sum of the fractions of the components a) to j) totals 100 % by weight.

2. The formulation according to claim 1, containing:
e) 1 - 2 % by weight of an antimicrobially acting glass filler, comprising 45 % by weight of SiO₂, 25 % by weight of Na₂O, 25 % by weight of CaO, and 5 % by weight of P₂O₅,
f) 0.01 - 1 % by weight of silver particles having a particle diameter < 30 nm.

3. The formulation according to claim 1 or 2, containing:
a) 20 - 75 % by weight of one or a plurality of aliphatic or cycloaliphatic monomeric/oligomeric urethane (meth)acrylates having a functionality n < 4, a viscosity < 15 Pas and a molecular weight < 2000,
b) 5 - 25 % by weight of a n-fold ethoxylated bisphenol A dimethacrylate having an ethoxylation degree > 10 or a mixture of n-fold ethoxylated bisphenol A dimethacrylates having an ethoxylation degree ≤ 30,
c) 2.5 - 10 % by weight of an n-fold cross-linking monomeric or oligomeric component, **characterized by** n ≥ 4 methacrylate and/or acrylate functions having a viscosity ≤ 20 Pas,
d) 4 - 15 % by weight of one or a plurality of monofunctional (meth)acrylates having a viscosity < 3 Pa s,
e) 1 - 2 % by weight of an antimicrobially acting glass filler, comprising 45 % by weight of SiO₂, 25 % by weight of Na₂O, 25 % by weight of CaO, and 5 % by weight of P₂O₅,
f) 0.01 - 1 % by weight of silver particles having a particle diameter < 30 nm,
g) 1 - 4.5 % by weight of one or a combination of a plurality of photoinitiators, the absorption of which is in the wavelength range of the laser beam or of the radiation source used,
h) 0 - 0.5 % by weight of one or a combination of anaerobic inhibitors, also in conjunction with aerobic inhibitors known to the man skilled in the art of stereo lithography,
i) 0 - 10 % by weight of fillers,
j) 0 - 4 % by weight of colorants,
k) 0.01 - 3 % by weight of conventional additives such as UV stabilizers or flow additives, wherein the sum of the fractions of the components a) to j) totals 100 % by weight.

## Revendications

1. Formulation durcissable par irradiation et à basse viscosité et biocompatible pour la préparation de produits médicaux avec des propriétés antimicrobiennes, en particulier d'embouts auriculaires au moyen de procédés PNP ou de procédés de fabrication génératifs, contenant:
a) 20 à 75 % en poids d'un ou de plusieurs (méth)acrylates d'uréthane aliphatiques ou cycloaliphatiques monomères/oligomères ayant une fonctionnalité n < 4, une viscosité < 15 Pa s et un poids moléculaire < 2000,
b) 5 à 25 % en poids d'un diméthacrylate de bisphénol A éthoxylé n fois, ayant un degré d'éthoxylation n > 10, ou d'un mélange de diméthacrylates de bisphénol A éthoxylés n fois, ayant un degré d'éthoxylation n ≤ 30,
c) 2,5 à 10 % en poids d'un composant monomère ou oligomère réticulant n fois, **caractérisé par** n ≥ 4 fonctions de méthacrylate et/ou acrylate ayant une viscosité ≤ 20 Pas,
d) 4 à 15 % en poids d'un ou de plusieurs (méth)-acrylates monofonctionnels ayant une viscosité < 3 Pa s,
e) 1 à 4 % en poids d'une matière de remplissage de verre ayant une action microbienne et consistant en 45 ± 5 % en poids de SiO₂, 25 ± 5 % en poids de Na₂O, 25 ± 5 % en poids de CaO et 5 ± 5 % en poids de P₂O₅, ou une combinaison d'additifs microbiens à partir des groupes des matières de remplissage de verre ayant une action microbienne ou des nanoparticules d'argent,
f) 1 à 4,5 % en poids d'un ou d'une combinaison de plusieurs photoamorceurs dont l'absorption se situe dans la plage de longueurs d'ondes du faisceau laser utilisé ou de la source d'irradiation utilisée,
g) 0 à 0,5 % en poids d'un ou d'une combinaison d'inhibiteurs anérobies, aussi associés à des inhibiteurs érobies connus à l'homme du métier de la stéréolithographie,
h) 0 à 10 % en poids de matières de remplissage,
i) 0 à 4 % en poids de colorants,
j) 0,01 à 3 % en poids d'additifs usuels tels que des agents de stabilisation aux UV ou des additifs d'écoulement, la somme des composants a) à j) étant égale à 100 % en poids.

2. Formulation selon la revendication 1, contenant:
e) 1 à 2 % en poids d'une matière de remplissage de verre ayant une action microbienne et consistant en 45 % en poids de SiO₂, 25 % en poids de Na₂O, 25 % en poids de CaO et 5 % en poids de P₂O₅,
f) 0,01 à 1 % en poids de particules d'argent ayant une taille de particule < 30 nm.

3. Formulation selon la revendication 1 ou 2, contenant:
a) 20 à 75 % en poids d'un ou de plusieurs (méth)acrylates d'uréthane aliphatiques ou cycloaliphatiques monomères/oligomères ayant une fonctionnalité n < 4, une viscosité < 15 Pa s et un poids moléculaire < 2000,
b) 5 à 25 % en poids d'un diméthacrylate de bisphénol A éthoxylé n fois, ayant un degré d'éthoxylation n > 10, ou d'un mélange de diméthacrylates de bisphénol A éthoxylés n fois, ayant un degré d'éthoxylation n ≤ 30,
c) 2,5 à 10 % en poids d'un composant monomère ou oligomère réticulant n fois, **caractérisé par** n ≥ 4 fonctions de méthacrylate et/ou acrylate ayant une viscosité ≤ 20 Pas,
d) 4 à 15 % en poids d'un ou de plusieurs (méth)-acrylates monofonctionnels ayant une viscosité < 3 Pa s,
e) 1 à 2 % en poids d'une matière de remplissage de verre ayant une action microbienne et consistant en 45 % en poids de SiO₂, 25 % en poids de Na₂O, 25 % en poids de CaO et 5 % en poids de P₂O₅,
f) 0,01 à 1 % en poids de particules d'argent ayant une taille de particule < 30 nm,
g) 1 à 4,5 % en poids d'un ou d'une combinaison de plusieurs photoamorceurs dont l'absorption se situe dans la plage de longueurs d'ondes du faisceau laser utilisé ou de la source d'irradiation utilisée,
h) 0 à 0,5 % en poids d'un ou d'une combinaison d'inhibiteurs anérobies, aussi associés à des inhibiteurs érobies connus à l'homme du métier de la stéréolithographie,
i) 0 à 10 % en poids de matières de remplissage,
j) 0 à 4 % en poids de colorants,
k) 0,01 à 3 % en poids d'additifs usuels tels que des agents de stabilisation aux UV ou des additifs d'écoulement, la somme des composants a) à j) étant égale à 100 % en poids.
